# EUROPÄISCHE PATENTSCHRIFT

(11) **EP 1 251 778 B1**
(45) Veröffentlichungstag und Bekanntmachung des Hinweises auf die Patenterteilung: **07.12.2005**
(21) Anmeldenummer: 00901027.3
(22) Anmeldetag: 02.02.2000
(51) Int. Cl.: A61B 5/06, A61N 5/10

(54) **VORRICHTUNG ZUR POSITIONSBESTIMMUNG VON KÖRPERTEILEN UND VERWENDUNG DER VORRICHTUNG**
DEVICE FOR DETERMINING THE POSITION OF BODY PARTS AND USE OF THE SAME
DISPOSITIF POUR DETERMINER LA POSITION DE PARTIES DU CORPS, ET UTILISATION DE CE DISPOSITIF

(43) Veröffentlichungstag der Anmeldung: 30.10.2002
(73) Patentinhaber: Northern Digital Inc., Waterloo, Ontario N2V 1C5 (CA)
(72) Erfinder: SEILER, Paul, G., CH-5232 Villigen (CH)
(74) Vertreter: Troesch Scheidegger Werner AG
(86) Internationale Anmeldenummer: PCT/CH2000/000055
(87) Internationale Veröffentlichungsnummer: WO 2000/022904

(56) Entgegenhaltungen:
- EP-A- 0 829 229
- WO-A-97/36192
- NL-C- 1 004 381
- US-A- 5 335 669
- US-A- 5 785 051
- US-A- 5 938 605

## Beschreibung

Die vorliegende Erfindung betrifft eine Vorrichtung zur Bestrahlung eines menschlichen oder tierischen Körpers.

Die moderne Strahlentherapie zur Behandlung von Tumoren zielt auf deren Zerstörung durch Applikation einer hohen, für den Tumor tödlichen Strahlendosis. Dies ist immer dann schwierig, wenn sich in unmittelbarer Nachbarschaft des Tumors Organe befinden, die nur wenig Strahlung tolerieren können (sogenannte Risikoorgane). Im Bauch- und Brustraum ergibt sich eine zusätzliche Schwierigkeit aus der Tatsache, dass sich dort Tumore und die benachbarten Risikoorgane im Körper des Patienten bewegen. Das geometrische Zielgebiet der Bestrahlung ist dann nicht ortsfest und es werden deshalb in diesen Fällen häufig benachbarte gesunde Organe oder Teile von ihnen unnötig stark bestrahlt. Die Ursachen der erwähnten Bewegungen sind die Lungenbewegung beim Atmen, der Herzschlag und die Darmtätigkeit. Positionen von Tumoren und Organen können sich auch auf Grund des sich verändernden Gesundheitszustandes des Patienten oder durch seine unterschiedliche Lagerung ändern.

Mit modernen Bestrahlungseinrichtungen kann heute im Körper eines Patienten ein dreidimensionales Gebiet (Hochdosisvolumen) intensiv so bestrahlt werden, dass seine Form genau die Form des Tumors nachbildet (konformale Radiotherapie). Besonders wichtig ist dabei, dass das Hochdosisvolumen benachbarte Risikoorgane ausschliesst (konformale Vermeidung).

In der konformalen Strahlentherapie der Prostata wird heute schon ein sogenannter Ballon-Katheter verwendet. Vor den Computertomographie-Aufnahmen, die der Planung der Strahlentherapie dienen, und vor jeder einzelnen Bestrahlung wird ein Katheter, der einen füllbaren, ausdehnbaren Katheterkopf besitzt, durch den After (Anus) in den Enddarm (Rectum) eingeführt und dann mit einer vorgegebenen Menge Wasser gefüllt. Dadurch wird die Prostata durch den ausgedehnten Katheterkopf gegen das Schambein gedrückt und damit bei den Bestrahlungen in eine einigermassen wiederholbare Position gebracht. Trotzdem erhält die der Prostata benachbarte Wand des Enddarmes eine nicht risikolose Strahlendosis, da die Position dieses Darmteiles nicht genügend gut bekannt ist. Die Forderung nach konformaler Vermeidung wird also nicht erfüllt.

Aus den Druckschriften EP-A-0 829 229, US-A-5 785 051 und NL-A-1 004 381 sind Verfahren und Vorrichtungen zur Positionsbestimmung und Positionskorrektur bekannt, die sich jedoch nicht für die konformale Vermeidung eignen.

Der vorliegenden Erfindung liegt daher die Aufgabe zugrunde, eine Vorrichtung anzugeben, mit der die konformale Vermeidung optimiert, d.h. gesteigert ist.

Diese Aufgabe wird durch die im Patentanspruchs 1 angegebenen Massnahmen gelöst. Vorteilhafte Ausgestaltungen der Erfindung sind in weiteren Ansprüchen angegeben.

Die Erfindung besteht in einem speziellen Katheter, der mindestens einen dehnbaren Teil aufweist, wobei erfindungsgemäss mindestens ein Sensor zur Bestimmung der Position, vorzugsweise auf der inneren Oberfläche eines der dehnbaren Teile vorgesehen ist.

In einer bevorzugten Ausführungsform der erfindungsgemässen Vorrichtung enthält der Katheter einen dehnbaren Teil, der gleichzeitig den Katheterkopf bildet. Im Falle von Strahlentherapie der Prostata wird der erfindungsgemässe Katheter - wie der aus dem Stand der Technik bekannte Ballon-Katheter - in den Enddarm eingeführt und in der Folge mit Wasser und/oder Luft gefüllt. Mit Hilfe des im Katheterkopf enthaltenen Sensors wird die Position in drei Koordinaten und die Ausrichtung in zwei Winkeln gemessen. Der Katheter ist so ausgebildet, dass sich der Sensor nach Positionierung und Füllung des Katheters an die Innenseite des Teiles der Darmwand anschmiegt, der der Prostata unmittelbar benachbart ist und der speziell vor einer riskanten Strahlendosis geschützt werden soll. Die ständige Überwachung der Sensorposition und -ausrichtung während der Bestrahlung kann, da sie mögliche Verlagerungen des Risikogebietes der Enddarmwand anzeigt, zur optimalen konformalen Vermeidung ihrer Bestrahlung eingesetzt werden. Entweder wird die Bestrahlung bei Wegbewegung der Darmwand (des Risikoorgans) unterbrochen und bei deren Rückkehr wieder aufgenommen oder das Bestrahlunsfeld wird kontinuierlich der Position von Tumor und Darmwand angepasst.

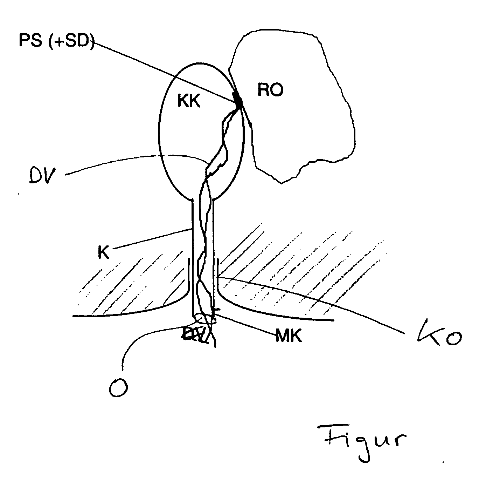

Die Erfindung wird nachfolgend anhand einer Zeichnung beispielsweise näher erläutert. Dabei zeigt die einzige Zeichnung in schematischer und ausschnittsweiser Darstellung einen erfindungsgemässen Katheter, der in einen Körperhohlraum eingeführt ist, wobei ein Katheterkopf zur Fixierung eines Körperteils gedehnt ist.

In der einzigen Figur ist eine erfindungsgemässe Vorrichtung zur häufig wiederkehrenden genauen Messung der Position gewisser gesunder Organe oder Teile von ihnen - im folgenden Risikoorgane RO genannt - zur Verhinderung ihrer Schädigung bei der Strahlentherapie unmittelbar benachbarter Tumoren dargestellt. Die erfindungsgemässe Vorrichtung besteht aus einem Katheter K, einem aus dehnbarem Material hergestellten Katheterkopf KK und einem Sensor PS zur Bestimmung der Position. Der Katheter K geht an seinem distalen Ende in den Katheterkopf KK über, wobei der Katheter K und der Katheterkopf KK, abgesehen von einer Öffnung O am diesseitigen Ende des Katheters K, eine luftdicht abgeschlossene Einheit bilden.

In der einzigen Figur ist die erfindungsgemässe Vorrichtung durch eine Körperöffnung KO in einen Hohlraum eingeführt dargestellt. Der Katheterkopf KK ist durch Zufuhr von Wasser und/oder Luft gedehnt, welche durch die Öffnung O zugeführt worden sind. Der auf der inneren Oberfläche des Katheterkopfes KK befestigte Sensor PS, mit dem die Position bestimmbar ist, wird gegen ein Risikoorgan RO gepresst, das eine möglichst geringe Strahlendosis erhalten soll.

Der Sensor PS erzeugt elektrische Signale. Diese werden entweder drahtlos oder über eine feine, im Inneren des Katheters K bzw. des Katheterkopfes KK verlaufende Drahtverbindung DV aus dem Körperinneren nach Aussen übertragen und in eine Empfangseinheit (in der Figur nicht dargestellt) eingespeist. Dort wird dann mit Hilfe von Hard- und Software mit einer hohen Frequenz - z.B 50 mal pro Sekunde - immer wieder die Position des Sensors PS und dessen Ausrichtung bestimmt. Es hat sich gezeigt, dass sich die in der WO 97/36 192 beschriebene Vorrichtung zur Bestimmung der Position besonders eignet.

Die erfindungsgemässe Vorrichtung eignet sich insbesondere zur Behandlung von Prostata. Die erfindungsgemässe Vorrichtung und dessen Verwendung lassen sich aber immer dann erfolgreich einsetzten, wenn natürliche Körperöffnungen (Nase, Mund, Darm, Scheide) in der konformalen Strahlentherapie und konformalen Bestrahlungsvermeidung benutzt werden können.

In einer weiteren Ausführungsform der Erfindung wird vorzugsweise unmittelbar neben dem Sensor PS zur Bestimmung der Position eine Dosimetereinheit SD integriert, mit der überprüft werden kann, ob die Strahlendosis im Risikoorgan RO so klein gehalten wird wie angestrebt.

Eine weitere Ausführungsform der Erfindung weist eine Markierung MK an einem Teil des Katheters K auf, der sich bei der Behandlung ausserhalb des Körpers des Patienten befindet. Die Markierung MK zeigt die Lage des Sensors PS bzw. der Dosimetereinheit SD bzgl. der Achse des Katheters K an. Diese Markierung MK dient dazu, den Katheter K richtig und nicht verdreht einzuführen.

In einer weiteren Ausführungsform der Erfindung sind zusätzliche Sensoren (in der Figur nicht dargestellt) auf der inneren und/oder auf der äusseren Oberfläche des Katheterkopfes KK vorgesehen. Beispielsweise können von einem O₂-Sensor (Sauerstoffsensor) für den Radiologen wichtige Informationen abgeleitet werden.

Obwohl ausdrücklich darauf hingewiesen worden ist, dass der Sensor PS zur Positionsbestimmung möglichst nahe an ein Risikoorgan RO, das nicht bzw. möglichst gering bestrahlt werden darf, plaziert werden soll, ist es, ausgehend von der hierin beschriebenen Lehre, eine einfache Anpassung der Verwendung der erfindungsgemässen Vorrichtung, wenn nunmehr der Sensor PS bzw. die Dosimetereinheit SD möglichst nahe an einen zu bestrahlenden Körperteil plaziert wird.

## Patentansprüche

1. Vorrichtung zur Bestrahlung eines menschlichen oder tierischen Körpers, wobei die Vorrichtung aus einem Katheter (K) zum Einführen in den Körper, aus mindestens einer Sensoreinheit (PS) zur Bestimmung der aktuellen Position, aus einer Bestrahlungseinheit und aus einer ausserhalb des Körpers angeordneten Empfangseinheit besteht, an welche die mit der Sensoreinheit (PS) gemessenen Signale übertragbar sind, wobei mindestens ein Teil des Katheters (K) dehnbar ist und wobei mindestens eine Sensoreinheit (PS) in dem mindestens einen dehnbaren Teil des Katheters (K) enthalten ist.

2. Vorrichtung nach Anspruch 1, **dadurch gekennzeichnet, dass** die mindestens eine Sensoreinheit (PS) an der inneren Oberfläche des Katheters (KK) angeordnet ist.

3. Vorrichtung nach Anspruch 1 oder 2, **dadurch gekennzeichnet, dass** der Katheter (K) einen einzigen dehnbaren Teil aufweist, wobei sich dieser Teil am Katheterkopf (KK) befindet.

4. Vorrichtung nach einem der Ansprüche 1 bis 3, **dadurch gekennzeichnet, dass** eine Dosimetereinheit (SD) vorgesehen ist, die sich im gleichen dehnbaren Teil (KK) befindet wie die Sensoreinheit (PS), und dass die mit der Dosimetereinheit (SD) gemessenen Signale vorzugsweise an die Empfangseinheit übertragbar sind.

5. Vorrichtung nach Anspruch 4, **dadurch gekennzeichnet, dass** die Dosimetereinheit (SD) unmittelbar neben der Sensoreinheit (PS) angeordnet ist.

6. Vorrichtung nach einem der vorangehenden Ansprüche, **dadurch gekennzeichnet, dass** eine Drahtverbindung (DV) durch den Katheter (K) zur Verbindung der Sensoreinheit (PS) und/oder der Dosimetereinheit (SD) mit der Empfangseinheit vorgesehen ist.

7. Vorrichtung nach einem der Ansprüche 4 bis 6, **dadurch gekennzeichnet, dass** die Bestrahlungseinheit mit Hilfe der Dosimetereinheit (SD) überwachbar ist.

8. Vorrichtung nach einem der vorangehenden Ansprüche, **dadurch gekennzeichnet, dass** eine Markierung (MK) am Katheter (K) angebracht ist.

9. Vorrichtung nach einem der vorangehenden Ansprüche, **dadurch gekennzeichnet, dass** die-Bestrahlungseinheit dem zu bestrahlenden Körperteil nachführbar ist.

10. Vorrichtung nach einem der Ansprüche 1 bis 8, **dadurch gekennzeichnet, dass** die Bestrahlungseinheit jeweils dann aktivierbar ist, wenn sich der zu bestrahlende Körperteil in einem durch die Bestrahlungseinheit vorgegeben Wirkungsbereich befindet.

## Claims

1. Device for the radiation of a human or animal body, the device comprising a catheter (K) for introduction into the body, at least one sensor unit (PS) for determining the current position, a radiation unit and a receiver unit provided outside the body, to which signals measured using the sensor unit (PS) can be transmitted, at least one part of the catheter (K) being expandable, and at least one sensor unit (PS) being contained in the at least one expandable part of the catheter (K).

2. Device according to claim 1, **characterized in that** the at least one sensor unit (PS) is arranged on the inner surface of the catheter (KK).

3. Device according to claim 1 or 2, **characterized in that** the catheter (K) has a single expandable part, the part being located on the catheter head (KK).

4. Device according to one of the claims 1 to 3, **characterized in that** a dose rate unit (SD) is provided, which is located in the same expandable part (KK) as the sensor unit (PS), and that the signals measured with the dose rate unit (SD) can preferably be transmitted to the receiver unit.

5. Device according to claim 4, **characterized in that** the dose rate unit (SD) is disposed directly adjacent to the sensor unit (PS).

6. Device according to one of the preceding claims, **characterized in that** a wire connection (DV) is provided through the catheter (K) for connecting the sensor unit (PS) and/or the dose rate unit (SD) to the receiver unit.

7. Device according to one of the claims 4 to 6, **characterized in that** the radiation unit is monitored using the dose rate unit (SD).

8. Device according to one of the preceding claims, **characterized in that** a marking (MK) is provided on the catheter (K)

9. Device according to one of the preceding claims, **characterized in that** the radiation unit follows the body part to be irradiated.

10. Device according to one of the claims 1 to 8, **characterized in that** the radiation unit is activated when the body part is located in a region of effectiveness, as predetermined by the radiation therapy unit.

## Revendications

1. Dispositif pour la radiothérapie d'un corps humain ou animal, le dispositif comprenant un cathéter (K) destiné à être introduit dans le corps, au moins une unité capteur (PS) pour déterminer la position actuelle, une unité d'irradiation et une unité récepteur disposée en dehors du corps et à laquelle les signaux mesurés avec l'unité capteur (PS) peuvent être transmis, au moins une partie du cathéter (K) étant dilatable et au moins une unité capteur (PS) étant installée dans ladite au moins une partie dilatable au cathéter (K).

2. Dispositif selon la revendication 1, **caractérisé en ce que** ladite au moins une unité capteur (PS) est disposée sur la surface intérieure du cathéter (KK).

3. Dispositif selon la revendication 1 ou 2, **caractérisé en ce que** le cathéter (K) présente une partie unique qui est dilatable, cette partie se trouvant dans la tête (KK) du cathéter.

4. Dispositif selon l'une des revendications 1 à 3, **caractérisé par** une unité dosimètre (SD) se trouvant dans la même partie dilatable (KK) que l'unité capteur (PS) et en ce que les signaux mesurés par l'unité dosimètre (SD) sont de préférence transmissibles à l'unité récepteur.

5. Dispositif selon la revendication 4, **caractérisé en ce que** l'unité dosimètre (SD) est disposée directement à côté de l'unité capteur (PS).

6. Dispositif selon l'une des revendications précédentes, **caractérisé par** une connexion par fils (DV) passant par le cathéter (K) est prévue pour connecter l'unité capteur (PS) et/ou l'unité dosimètre (SD) avec l'unité récepteur.

7. Dispositif selon l'une des revendications précédentes, **caractérisé en ce que** l'unité d'irradiation peut être surveillée à l'aide de l'unité dosimètre (SD).

8. Dispositif selon l'une des revendications 4 à 6, **caractérisé en ce qu'**un repère (MK) est disposé sur le cathéter (K).

9. Dispositif selon l'une des revendications précédentes, **caractérisé en ce que** l'unité d'irradiation peut être pointée sur la partie du corps à irradier.

10. Dispositif selon l'une des revendications 1 à 8, **caractérisé en ce que** l'unité d'irradiation peut être activée chaque fois que la partie du corps à irradier se trouve dans une joue active prédéterminée par l'unité d'irradiation.
